# EUROPEAN PATENT SPECIFICATION

(11) **EP 2 195 113 B1**
(45) Date of publication and mention of the grant of the patent: **04.03.2020**
(21) Application number: 08828969.9
(22) Date of filing: 03.09.2008
(51) Int. Cl.: B01L 3/00, C12Q 1/42, G01N 30/88

(54) **MICROFLUIDIC METHOD AND SYSTEM FOR ENZYME INHIBITION ACTIVITY SCREENING**
MIKROFLUIDISCHES VERFAHREN UND SYSTEM ZUM SCREENING VON ENZYMHEMMUNGSAKTIVITÄT
PROCÉDÉ ET SYSTÈME MICROFLUIDIQUE DE CRIBLAGE À LA RECHERCHE D'UNE ACTIVITÉ D'INHIBITION ENZYMATIQUE

(30) Priority: 06.09.2007 US 899599
(43) Date of publication of application: 16.06.2010
(60) Divisional application: 20153233.0
(73) Proprietor: Caliper Life Sciences, Inc., Mountain View, CA 94043 (US)
(72) Inventor: DUNNE, Jude, A., Menlo Park, CA 94025 (US); FARINAS, Javier, A., Los Altos, CA 94024 (US); KAZAKOVA, Irina, G., Los Gatos, CA 95032 (US); HUANG, Esther, Mountain View, CA 94043 (US); GERBER, Raphaele, Foster City, CA 94404 (US); HACKER, Coleen, Cupertino, CA 95014 (US); DETTLOFF, Roger, Emerald Hills, CA 94062 (US); TRINH, Thi Ngoc Vy, Cupertino, CA 95014 (US)
(74) Representative: Mewburn Ellis LLP
(86) International application number: PCT/US2008/075117
(87) International publication number: WO 2009/032842

(56) References cited:
- WO-A-00/72016
- WO-A-00/75167
- WO-A-96/04547
- US-A1- 2005 221 385

## Description

### FIELD OF THE INVENTION

The present invention relates to kits, systems, and methods for determining the level of enzyme activity in the presence of a compound, and more particularly to kits, systems, and methods for identifying drugs that modulate kinase activity across an array of kinases.

### BACKGROUND OF THE INVENTION

Kinases are enzymes that catalyze the transfer of a phosphate group from ATP (adenosine triphosphate) or another nucleoside triphosphate to a substrate. For example, hexokinase catalyzes the phosphoryl transfer from ATP to glucose to produce glucose-6-phosphate as an initial step in glycolysis. Other kinases involved in glycolysis include phosphofructokinase, phosphoglycerate kinase, and pyruvate kinase. Kinases are also involved, e.g., in protein phosphorylation by transferring the terminal phosphate from ATP to a side chain of an amino acid residue of a protein. In eukaryotic cells, protein phosphorylation serves various functions including, e.g., the phosphorylation of cell-surface receptors to produce intracellular effects, the regulation of the cell cycle, the protection of cells from toxic changes in metabolites, or the like.

Protein kinases comprise a large family of enzymes with over 500 different forms identified to date in the human genome. Protein substrates for these enzymes may include up to one-third of all cellular proteins. An understanding of the protein kinase enzymes and their targets is crucial to understanding cellular regulation and cellular pathology.

Protein kinases play very important roles in many cell functions, including, but not limited to, cellular metabolism, signal transduction, transcriptional regulation, cell motility, cell division, cellular signaling processes, cellular proliferation, cellular differentiation, apoptosis, and secretion. These processes are mediated by phosphorylation or dephosphorylation of enzymes, protein substrates, transcription factors, hormone or growth factor receptors, and other cellular proteins. Protein phosphorylation is a regulatory mechanism used by cells to selectively modify proteins carrying regulatory signals from outside the cell to the nucleus.

Protein kinases are also involved in mediating the response to naturally occurring toxins and pathogens, which alter the phosphorylation states of proteins. Additionally, protein kinases are related to many epidemiologically relevant oncogenes and tumor suppressor genes. In fact, protein kinases are implicated in several hundred human diseases. Examples include neurodegenerative diseases such as amyotrophic lateral sclerosis and Alzheimer's disease. Consequently, protein kinases are often validated drug targets since human diseases are frequently linked to dysregulation of cellular signaling pathways.

With phosphorylation involved in so many cell functions and diseases, identifying compounds that affect protein kinase activity is tremendously important, not only to provide drug candidates that are active in treating disease, but also to ensure that these drug candidates act without causing adverse effects on other cellular functions. Thus, there is a need for high-throughput screening assays to identify activators and inhibitors of kinases as well as kits for carrying out such assays.

WO 00/75167 describes assays for detecting phosphorylation and dephosphorylation modifications of proteins and the presence, activity, and modulation of enzymes effecting these modifications, including kinases and phosphatases, respectively.

### SUMMARY OF THE INVENTION

One aspect of the present invention is a kit according to claim 1 for screening a compound for enzyme inhibition activity. The kit includes first and second multiwell plates. The first multiwell plate has a plurality of enzymes disposed within a first plurality of wells. The second multiwell plate has a plurality of enzyme substrates disposed within a second plurality of wells. A phosphate source is disposed within each well of the second plate, the phosphate source disposed in the well at a predetermined concentration. In addition, a cofactor is disposed within each well of the second plate, the cofactor disposed in each well at a predetermined concentration.

Another aspect of the present invention is a system according to claim 3 for screening a compound for enzyme inhibition activity.
The system includes a kit according to claim 1 or 2 and a microfluidic device. The microfluidic device includes at least one microchannel and a capillary element, the capillary element operably connected to and in fluid communication with the microchannel. A detector may be operably connected to the microfluidic device. The system may further include a computer. A controller and a fluid direction system are operably connected to the computer The fluid direction system includes a pressure source in fluid communication with the microfluidic device and a sample source, the sample source in fluid communication with the capillary element.
Yet another aspect of the present invention is a method according to claim 5 for screening a compound for enzyme inhibition activity.
A sample mixture comprising an enzyme, an enzyme substrate, a test compound, and a product is prepared using a kit according to claim 1 or 2 The sample mixture is provided to a microfluidic device. Vacuum pressure is applied to the sample mixture, flowing the sample mixture along a microchannel of the microfluidic device. The sample mixture is separated based upon a difference in electrophoretic mobility between the product and the enzyme substrate. The separated product and enzyme substrate are detected, and enzyme inhibition activity is determined based on the detection of the separated product and enzyme substrate.

Many additional aspects of the invention will be apparent upon review, including uses of the devices and systems of the invention, methods of manufacture of the devices and systems of the invention, kits for practicing the methods of the invention, and the like. For example, kits comprising any of the devices or systems set forth above, or elements thereof, in conjunction with packaging materials (e.g., containers, sealable plastic bags, etc.) and instructions for using the devices, e.g., to practice the methods herein, are also contemplated.

The present invention is illustrated by the accompanying drawings of various embodiments and the detailed description given below. The drawings should not be taken to limit the invention to the specific embodiments, but are for explanation and understanding. The detailed description and drawings are merely illustrative of the invention rather than limiting, the scope of the invention being defined by the appended claims.

### BRIEF DESCRIPTION OF THE DRAWINGS

**FIG. 1** schematically illustrates a system for screening a compound for enzyme inhibition activity, in accordance with the present invention;
**FIG. 2** illustrates one embodiment of an enzyme plate used in accordance with the present invention;
**FIG. 3** is a table illustrating components and reaction conditions for one embodiment of a screening kit, in accordance with the present invention;
**FIG. 4** is a table illustrating components and reaction conditions for another embodiment of a screening kit, in accordance with the present invention;
**FIG. 5** schematically illustrates a portion of a microfluidic device such as is illustrated in **FIG. 1****,** in accordance with the present invention;
**FIG. 6** illustrates enzyme degradation data for ABL;
**FIG. 7** illustrates the change in conversion per week for a subset of enzymes that was tested for degradation over 22 weeks (error bars = 95% confidence intervals);
**FIG. 8** illustrates selectivity data for staurosporine;
**FIG. 9** illustrates selectivity data for K252A; and
**FIG. 10** illustrates selectivity data SB203580.

The drawings are not to scale.

### DETAILED DESCRIPTION

Before describing the present invention in detail, it is to be understood that this invention is not limited to particular compositions, devices, or systems, which can, of course, vary. It is also to be understood that the terminology used herein is for the purpose of describing particular embodiments only and is not intended to be limiting. Further, unless defined otherwise, all technical and scientific terms used herein have the same meaning as commonly understood by one of ordinary skill in the art to which the invention pertains.

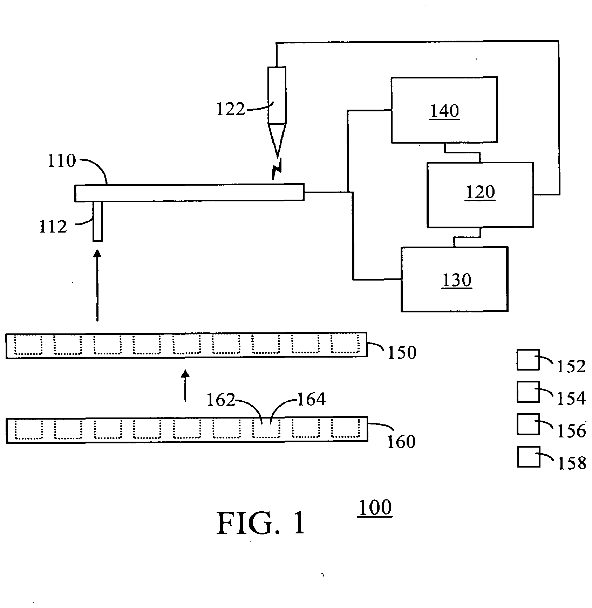

One aspect of the present invention is a kit for screening a compound for enzyme inhibition activity. In one embodiment, the kit includes first and second multiwell plates, i.e., enzyme plate **150** and substrate plate **160,** a reconstitution buffer **152,** DTT (dithiothreitol) **154,** a protease inhibitor **156,** and a termination buffer **158,** all of which components are included in the system illustrated at **100** in **FIG. 1****.** A plurality of enzymes are disposed within the wells of enzyme plate **150,** and a plurality of enzyme substrates, a phosphate source **162,** and a cofactor **164** are disposed within the wells of substrate plate **160.**

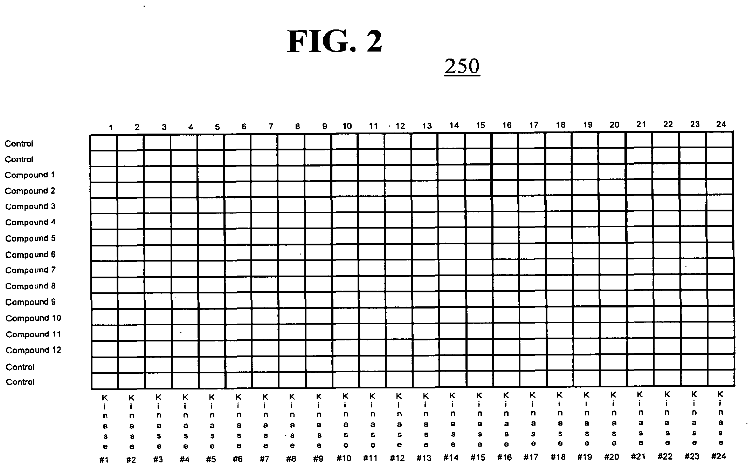

In one embodiment, enzyme plate **150** is an enzyme plate such as is illustrated in **FIG. 2** at **250.** Enzyme plate **250** includes a plurality of enzymes disposed within individual wells of plate **250.** The arrangement of the enzymes on the enzyme plate is determined by the positioning of the corresponding substrates on the substrate plate. Positioning of the substrates is based on factors such as, for example, electrophoretic mobility.

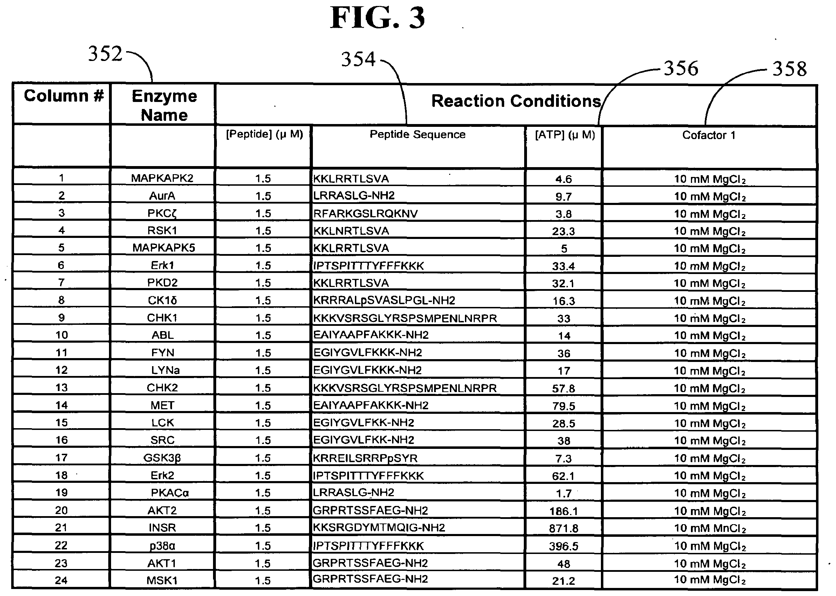

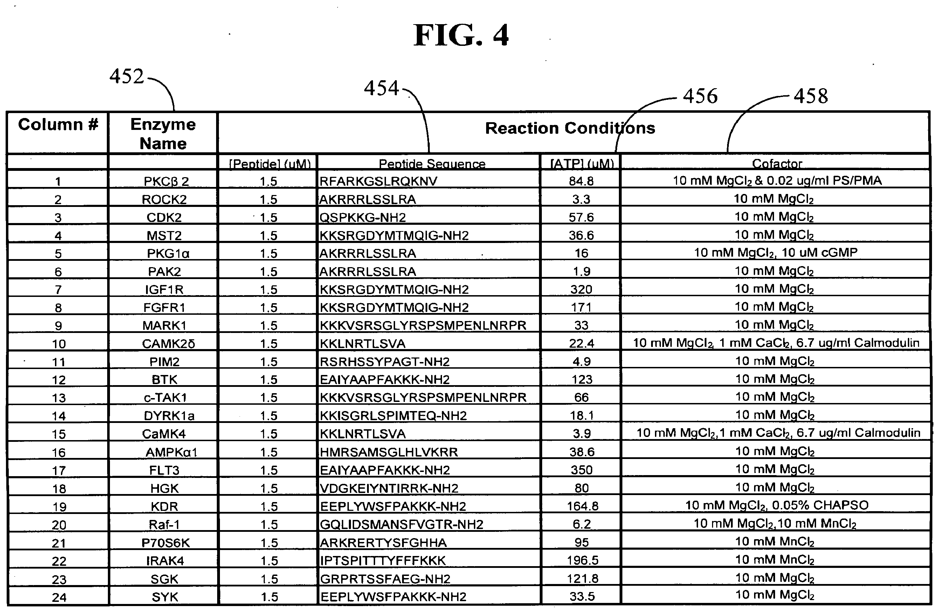

In this first illustrative embodiment, enzyme plate **250** includes a plurality of kinases numbered 1 through 24 disposed in columns 1 through 24 of plate **250.** Kinases 1 through 24 comprise enzymes **352** illustrated in **FIG. 3****.** Enzymes **352** include kinases MAPKAPK2, AurA, PKCζ, RSK1, MAPKAPK5, Erk1, PKD2, CK1δ, CHK1, ABL, FYN, LYNa, CHK2, MET, LCK, SRC, GSK3β, Erk2, PKACα, AKT2, INSR, p38α, AKT1, and MSK1. In this first embodiment, enzymes **352** are disposed within columns 1 through 24 in the exact order illustrated in **FIG. 3****.** In a second illustrative embodiment, kinases 1 through 24 comprise enzymes **452** illustrated in **FIG. 4****.** Enzymes **452** include kinases PKCβ2, ROCK2, CDK2, MST2, PKG1α, PAK2, IGF1R, FGFR1, MARK1, CAMK2δ, PIM2, BTK, c-TAK1, DYRK1a, CaMK4, AMPKα1, FLT3, HGK, KDR, Raf-1, P70S6K, IRAK4, SGK, and SYK. In this second embodiment, enzymes **452** are disposed within columns 1 through 24 in the exact order illustrated in **FIG. 4****.** While 24 columns of kinases are illustrated in **FIGS. 1-3****,** this is not intended to limit the invention. Other enzymes may be used, and the number of columns may be other than 24. The enzyme plate may also include a plurality of control wells as determined by the particular application.

Substrate plate **160** includes a plurality of enzyme substrates to be added to the enzyme plate. Each well of substrate plate **160** includes a solution containing a peptide, ATP (adenosine 5'-triphosphate), and a cofactor. Each enzyme acts on a specific peptide sequence. **FIGS. 3** and 4 illustrate the peptide sequences **354, 454** for each corresponding enzyme **352, 452,** respectively. **FIGS. 3** and **4** also illustrate the specific concentration of ATP **356, 456** and the specific cofactor and cofactor concentration **358, 458** required for each enzyme **352, 452.**

Kits in accordance with the present invention typically include all components necessary for screening a compound for enzyme inhibition activity, with the exception, of course, of the compound(s) of interest to be tested. For example, the kit may contain controls as indicated in **FIG. 2****,** a reconstitution buffer **152,** DTT **154** for addition to the reconstitution buffer prior to addition of the buffer to the enzyme plate, a protease inhibitor **156** also for addition to the reconstitution buffer prior to addition of the buffer to the enzyme plate, and a termination buffer **158** that is added to the enzyme plate to terminate the enzyme reaction prior to reading of the plate.

The enzymes used in the present invention are as defined in claim 1.

**FIGS. 3** and **4** provide examples of two kinase panels particularly suitable for use in the present invention.

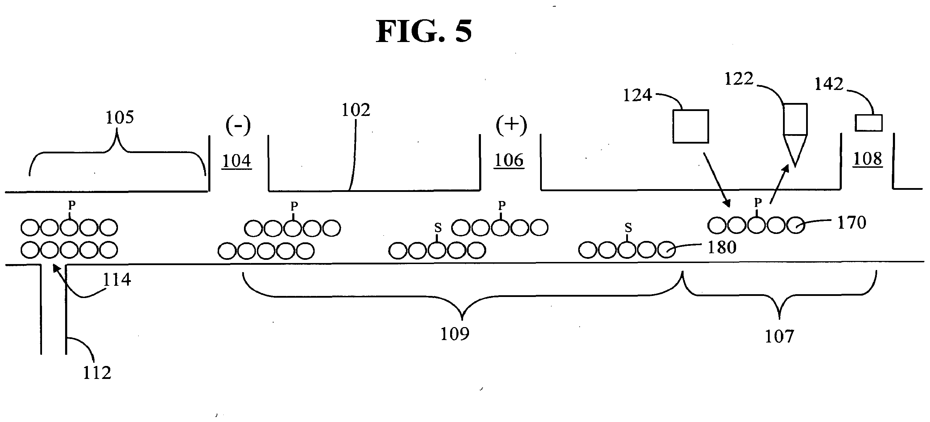

Another aspect of the invention is a system for screening a compound for enzyme inhibition activity. **FIGS. 1** and **5****,** which are not to scale, illustrate one embodiment of a system **100** for screening compounds for enzyme activity, e.g., protein kinase activity, in accordance with the present invention. Note that the system is also suitable for use with other enzymes such as defined in the claims.

System **100** includes a microfluidic device **110,** a computer **120,** a detector **122,** a controller **130,** and a fluid direction system **140.** The system additionally includes a kit such as has been described above, including first and second multiwell plates **150** and **160,** reconstitution buffer **152,** DTT **154,** protease inhibitor **156,** and termination buffer **158.**

Microfluidic device **110** includes at least one microchannel **102** and a plurality of reservoirs or ports **104, 106, 108** in fluid communication with microchannel **102.** Aliquots of a sample containing a test compound (prepared in first and second multiwell plates **150** and **160**) are flowed into main microchannel **102** from capillary element **112** towards reservoir **108** by, for example, applying a vacuum at reservoir **108** (or another point in the system) and/or by applying appropriate voltage gradients. As used herein, a "capillary element" includes an elongated body structure having a channel (e.g., a microchannel) disposed therethrough. A capillary element is preferably an integral extension of the body structure of a single microfluidic device, as illustrated in **FIG. 1****,** but may also be a separate component that is temporarily coupled to one or more microfluidic device body structures. A microfluidic device in accordance with the present invention may include a single capillary element as illustrated in **FIG. 1** or may include a plurality of capillary elements (e.g., 4 or 12).

Additional materials, such as buffer solutions, substrate solutions, enzyme solutions, and the like, are optionally flowed from reservoirs **104** or **106** and into main microchannel **102.** Flow from these reservoirs is optionally performed by modulating fluid pressure, by electrokinetic approaches, or both (as illustrated in **FIG. 5**). A number of possible arrangements of channels within microfluidic device **110** are possible, the arrangement depending on the specific application. Channels within the microfluidic device are covered, as detailed below under the heading "Microfluidic Devices."

Detector **122** is in sensory communication with main microchannel **102,** detecting signals resulting, for example, from labeled materials flowing through the detection region **107.** Detector **122** is optionally in sensory communication with any of the channels or regions of the device where detection is desired. As used herein, the phrase "in sensory communication" refers to positioning of a detector such that it is operably connected to the channel, i.e., capable of receiving a detectable signal from the contents of a channel. In the case of optical signals, this requires only that the detector be positioned to receive the optical signal. Detector **122** is also operably connected to computer **120,** which digitizes, stores, and manipulates signal information detected by detector **122,** for example, using any instruction set for determining enzyme activity, concentration, molecular weight or identity, or the like.

Fluid direction system **140** controls pressure, voltage, or both, e.g., at the reservoirs of the system or through the channels of the system, or at vacuum couplings fluidly coupled to main microchannel **102** or other channels described above. Optionally, as depicted, computer **120** controls fluid direction system **140.** In one set of embodiments, computer **120** uses signal information to select further parameters for the microfluidic system. In certain embodiments, controller **130** dispenses aliquots of buffers or other materials into, for example, main microchannel **102.** In these embodiments, controller **130** is also operably connected to computer **120,** which directs controller **130** function.

Although not shown, a microfluidic device handler is also typically included in the integrated systems of the present invention. Microfluidic device handlers generally control, e.g., the x-y-z translation of microfluidic device **110** relative to multiwell plate **150** or other system components, under the direction of computer **120,** to which the device handlers are operably connected. Microfluidic device handlers may also be involved in the transfer of materials from multiwell plate **160** to multiwell plate **150.** Microfluidic devices, computers, detectors, controllers, and fluid flow systems in accordance with the present invention are described in more detail below.

Yet another aspect of the present invention provides a method of screening a compound for enzyme inhibition activity. A sample mixture is obtained using a kit such as has been described above, the sample mixture comprising an enzyme, an enzyme substrate, a test compound and a product. The sample mixture is provided to a microfluidic device such as has been described above. Vacuum pressure is applied to the sample mixture, flowing the sample mixture along a microchannel of the microfluidic device, thereby separating the sample mixture to produce separated materials. The separated materials are detected, and enzyme inhibition activity is determined based on the detection of the separated materials.

The compounds to be screened are prepared using a multiwell compound plate in addition to enzyme and substrate plates such as those illustrated at **150** and **160,** respectively. The enzyme and substrate plates are stored frozen and are thawed prior to use.

Prepared compounds are added to enzyme plate **150** from the compound plate and incubated for a predetermined length of time as determined by the specific application. Reconstitution buffer **152,** combined with DTT **154** and a protease inhibitor **156,** is added to the enzyme plate either before adding the prepared compounds or in combination with the compounds. After expiration of the predetermined length of time, a portion of a substrate solution corresponding with a specific enzyme is removed from a well of substrate plate **160** and added to the corresponding well of enzyme plate **150.** This mixture is left to incubate for a predetermined length of time. After expiration of this time, termination buffer **158** is added to each well of enzyme plate **150.** See **Example 1,** below, for detailed steps. Aliquots of sample from each well are then provided to microfluidic device **110** for separation and analysis.

Each sample is flowed into region **105** of microchannel **102** via capillary element **112** by applying negative fluid pressure to the materials via a vacuum source applied to port **108.** The materials are flowed into a microchannel separation region **109.** Under an applied voltage, phosphorylated product is separated from unreacted substrate based upon a difference in mobilities of the phosphorylated product and the unreacted substrate. The separation region preferably contains free solution but may include an ion-exchange material (e.g., a polyarginine, a polylysine, a modified polyacrylamide, a modified dimethylacrylamide, or the like). Optionally, the ion-exchange material includes a polyacrylamide or a dimethylacrylamide modified (e.g., via covalent attachment, adsorption, or the like) by one or more anionic or cationic additives. In certain embodiments, the ion-exchange material is covalently or otherwise attached to a plurality of microbeads or a gel. In addition, the methods optionally include flowing the ion-exchange material into the separation region. The methods include detecting the resulting separated product and the unreacted substrate.

The sample is flowed through microchannel **102** and past detector **122.** As the sample nears detector **122,** a beam of light from LED (or laser or other light source) **124** is directed towards the sample. Fluorescence from the sample is then detected by detector **122.** Signals from detector **122** are transmitted to computer **120** for analysis and determination of the level of enzyme inhibition activity, if any, caused by the compound of interest.

In certain embodiments, more than one product is formed by contacting the first and second materials. These are also optionally separated according to the methods described herein. The methods optionally include flowing materials in the microfluidic device in the absence of an applied electric field, or flowing materials in the microfluidic device under a simultaneously applied electric field.

**FIG. 5** schematically illustrates one embodiment of an ion-exchange-induced mobility-shift-based separation method that is used for high-throughput compound screening in accordance with the present invention. As shown, microchannel configuration **100** includes microchannel **102** into which a sample including enzyme and substrate solutions is placed via capillary element **112.** Under negative pressure applied by a vacuum **142** at vacuum port **108,** the sample is continuously flowed from sample port **114** of main microchannel **102** towards port **108** and past detector **122** to detect a reaction product **170** and unreacted substrate **180.** Optionally, a modulator, an inhibitor, an antagonist, an agonist, or other material may be flowed from a well on a multiwell plate or another external source through capillary element **112** into region **105** of main microchannel **102,** e.g., to assess its impact on the assay.

A free solution or a solution of a suitable ion exchanger or other chromatographic material, as appropriate, is optionally placed into microchannel **102** and flowed continuously into separation region **109** of main microchannel **102** during the course of the assay to effect separation of the reaction product from unreacted substrate based upon their, e.g., respective mobilities or other distinguishing properties. Alternatively, separation region **109** of main microchannel **102** is selectively modified by, for example, coating it with the ion exchanger prior to commencing the assay. An additional option includes manufacturing at least separation region **109** of main microchannel **102** to possess the desired ion-exchange characteristics (e.g., by selecting appropriate microfluidic device substrate materials).

The method of screening a compound for enzyme inhibition activity includes mixing an enzyme solution (e.g., a protein kinase, a protein kinase A, a nucleic acid kinase, or the like) and a substrate solution to produce a phosphorylated product such as product **170** illustrated in **FIG. 5****.**

### EXAMPLE 1

The following example serves to illustrate, but not to limit, the present invention.

### Materials

A DeskTop Profiler™ instrument (available from Caliper Life Sciences, Inc.); ProfilerPro™ enzyme and substrate plates (available from Caliper Life Sciences, Inc.); a 384-well plate for control/compound preparation; reconstitution buffer, ATP/peptides, termination buffer, DTT, protease inhibitor, and DMSO; and microfluidic devices for performing mobility shift assays were used. The 384-well ProfilerPro™ enzyme plates include 24 enzymes per plate, pre-dispensed in columns of 16. The 384-well ProfilerPro™ substrate plates include 24 peptide/ATP pairs per plate, pre-dispensed in columns of 16. Prior to use, the plates are stored below -70 °C. Buffers are stored below 4 °C. DTT and protease inhibitor are stored below -70 °C.

### Procedure

1. Prepare components.
   a. Remove reconstitution buffer and termination buffer from freezer and thaw. If stored for extended periods prior to use, maintain at 4 °C.
   b. To 50 mL reconstitution buffer, add 50 µL of 1M DTT and 500 µL of 100x protease inhibitor.
   c. Prepare compounds.
      i. For 1 µL compound transfer, dilute compounds to 26x assay concentration in 100% DMSO; place in compound plate in rows C through N (see FIG. 2).
         **OR**
      ii. For 16 µL compound transfer, dilute compounds to 1.625x assay concentration in previously prepared reconstitution buffer with DTT and protease inhibitor; place in compound plate in rows C through N (see FIG. 2).
   d. Prepare controls.
      i. For 1 µL control transfer, add 100% DMSO to compound plate in rows A, B, O, and P (see FIG. 2).
         **OR**
      ii. For 16 µL control transfer, make 6.25% DMSO solution in previously prepared reconstitution buffer with DTT and protease inhibitor; place in compound plate in rows A, B, O, and P (see FIG. 2).
   e. Place reconstitution buffer, compound plate, and termination buffer in incubator at 28 °C.
2. Prepare ProfilerPro™ substrate and enzyme plates.
   a. Remove substrate plate from freezer and incubate at 28 °C.
   b. Wait 30 minutes
   c. Remove enzyme plate from freezer and incubate at 28 °C; after 15 minutes, spin enzyme plate at 1000 rpm for 1 minute if bubbles are observed; remove seal.
   d. Transfer compound/control from the compound plate to the enzyme plate.
      i. For 1 µL compound/control transfer, first add 15 µL of previously prepared reconstitution buffer with DTT and protease inhibitor to each well of the enzyme plate and mix; then add 1 µL compound/control from each well of the compound plate to a separate well of the enzyme plate; mix.
         **OR**
      ii. For 16 µL compound/control transfer, add 16 µL of the previously prepared mixture of compound/control and reconstitution buffer (with DTT and protease inhibitor) to each well of the enzyme plate and mix.
   e. Pre-incubate the enzyme plate for up to 15 minutes at 28 °C.
   f. At least 60 minutes after placing the substrate plate in the incubator in Step a, remove the substrate plate from the incubator and spin at 500 rpm for 1 minute if bubbles are observed; remove seal.
   g. Transfer 10 µL of substrate from each well of the substrate plate to each well of the enzyme plate; spin at 1000 rpm for 1 minute if bubbles are observed.
   h. Cover enzyme plate and incubate for 90 minutes at 28 °C.
   i. Add 45 µL of termination buffer to each well of the enzyme plate in the same order used when adding substrate (to avoid variable incubation time); mix if necessary; spin at 1000 rpm for 1 minute.
3. Read the enzyme plate using the DeskTop Profiler™ instrument.
   All assays are run at ATP/Km =1 for each enzyme. Common buffers are used:
   *Reaction Buffer*
      100 mM HEPES, pH = 7.5
      10 mM MgCl₂ or MnCl₂
      0.003% Brij-35
      1 mM DTT
      4% DMSO
      [E] is predetermined to yield the appropriate conversion upon incubation and termination.
   *Separation Buffer*
      100 mM HEPES, pH = 7.5
      0.015% Brij-35
      1 mM EDTA
      0.1 % Coating Reagent 3
      5% DMSO

### Results

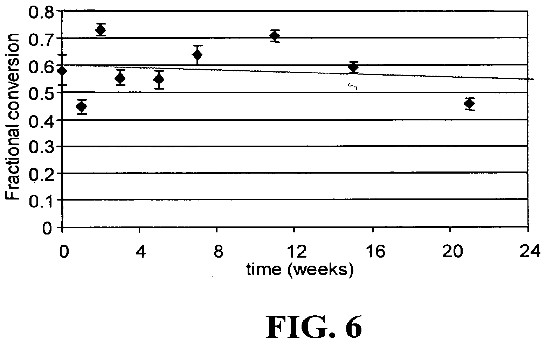

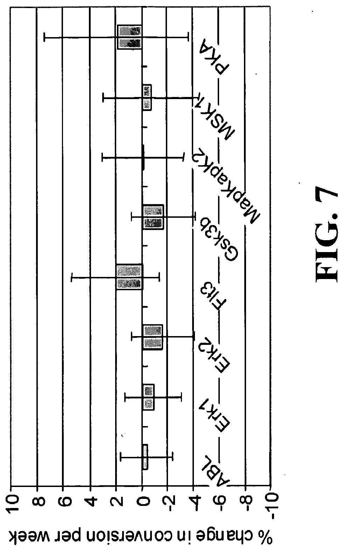

*Enzyme degradation.* Enzymes were diluted to 260x the assay concentration in a storage buffer and 100 nL dispensed into the wells of a 384-well microtiter plate. Plates were frozen and stored at -80 °C. Enzymes were assayed over 22 weeks by thawing and adding 16 µL of reconstitution buffer and 10 µL of substrate. After a 1-hour incubation, the reactions were quenched and run on a Caliper Life Sciences, Inc., LabChip® 3000 Drug Discovery System. Sample data are shown for ABL in **FIG. 6****.** **FIG. 7** shows the change in conversion per week for a subset of the enzymes that was tested for 22 weeks (error bars = 95% confidence intervals). No enzyme showed a statistically significant difference from no change in activity.

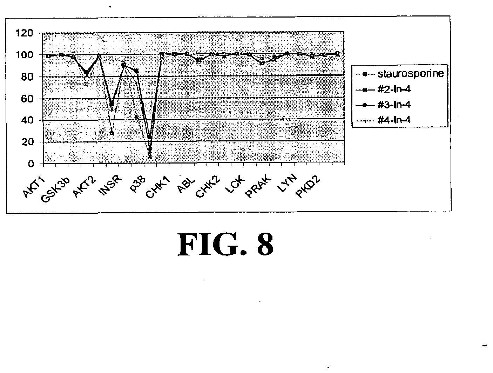

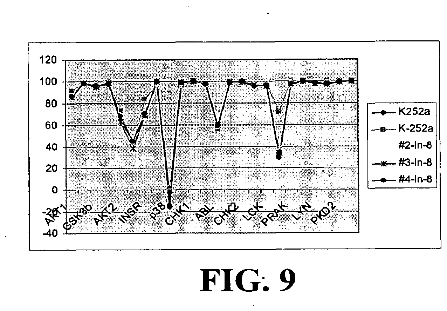

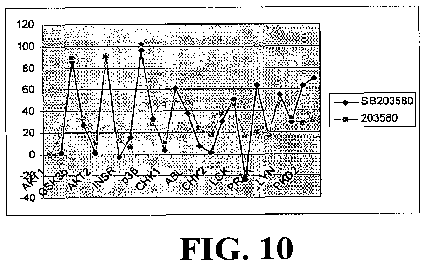

*Kinase Selectivity Screening.* A set of 13 commercially available kinase inhibitors was run at a concentration of 10 µM against a ProfilerPro™ enzyme plate. In this example, the enzymes were disposed within columns 1 through 24 in the order of AKT1, MSK1, GSK3β, Erk1, AKT2, CK1δ, INSR, Erk2, p38, MET, CHK1, SRC, ABL, RSK1, CHK2, FYN, LCK, PKCζ, PRAK, AurA, LYN, MAPKAPK2, PKD2, PKA. Selectivity data for staurosporine (**FIG. 8**), K252A (**FIG. 9**), and SB203580 (**FIG. 10**) were run at Caliper Life Sciences, Inc. and at an external site, showing good agreement.

*Reproducibility.* Two ProfilerPro™ plates from two batches were run each of three days with DMSO controls in 5 wells. An ANOVA revealed an overall CV of 18.8% with the following sources of variability (% of total):

| | |
|---|---|
| day-to-day | 13.9% |
| batch-to-batch | 0% |
| plate-to plate | 26.5% |
| assay | 59.6% |

### MICROFLUIDIC DEVICES

Many different microscale systems are optionally adapted for use in the methods of the present invention. These systems are described in numerous publications by the inventors and their coworkers, including certain issued U.S. Patents, such as U.S. Patent Nos. 5,699,157 (J. Wallace Parce) issued 12/16/97, 5,779,868 (J. Wallace Parce et al.) issued 07/14/98, 5,800,690 (Calvin Y.H. Chow et al.) issued 09/01/98, 5,842,787 (Anne R. Kopf-Sill et al.) issued 12/01/98, 5,852,495 (J. Wallace Parce) issued 12/22/98, 5,869,004 (J. Wallace Parce et al.) issued 02/09/99, 5,876,675 (Colin B. Kennedy) issued 03/02/99, 5,880,071 (J. Wallace Parce et al.) issued 03/09/99, 5,882,465 (Richard J. McReynolds) issued 03/16/99, 5,885,470 (J. Wallace Parce et al.) issued 03/23/99, 5,942,443 (J. Wallace Parce et al.) issued 08/24/99, 5,948,227 (Robert S. Dubrow) issued 09/07/99, 5,955,028 (Calvin Y.H. Chow) issued 09/21/99, 5,957,579 (Anne R. Kopf-Sill et al.) issued 09/28/99, 5,958,203 (J. Wallace Parce et al.) issued 09/28/99, 5,958,694 (Theo T. Nikiforov) issued 09/28/99, 5,959,291 (Morten J. Jensen) issued 09/28/99, 5,964,995 (Theo T. Nikiforov et al.) issued 10/12/99, 5,965,001 (Calvin Y. H. Chow et al.) issued 10/12/99, 5,965,410 (Calvin Y. H. Chow et al.) issued 10/12/99, 5,972,187 (J. Wallace Parce et al.) issued 10/26/99, 5,976,336 (Robert S. Dubrow et al.) issued 11/2/99, 5,989,402 (Calvin Y. H. Chow et al.) issued 11/23/99, 6,001,231 (Anne R. Kopf-Sill) issued 12/14/99, 6,011,252 (Morten J. Jensen) issued 1/4/00, 6,012,902 (J. Wallace Parce) issued 1/11/00, 6,042,709 (J. Wallace Parce et al.) issued 3/28/00, 6,042,710 (Robert S. Dubrow) issued 3/28/00, 6,046,056 (J. Wallace Parce et al.) issued 4/4/00, 6,048,498 (Colin B. Kennedy) issued 4/11/00, 6,068,752 (Robert S. Dubrow et al.) issued 5/30/00, 6,071,478 (Calvin Y. H. Chow) issued 6/6/00, 6,074,725 (Colin B. Kennedy) issued 6/13/00, 6,080,295 (J. Wallace Parce et al.) issued 6/27/00, 6,086,740 (Colin B. Kennedy) issued 7/11/00, 6,086,825 (Steven A. Sundberg et al.) issued 7/11/00, 6,090,251 (Steven A. Sundberg et al.) issued 7/18/00, 6,100,541 (Robert Nagle et al.) issued 8/8/00, 6,107,044 (Theo T. Nikiforov) issued 8/22/00, 6,123,798 (Khushroo Gandhi et al.) issued 9/26/00, 6,129,826 (Theo T. Nikiforov et al.) issued 10/10/00, 6,132,685 (Joseph E. Kersco et al.) issued 10/17/00, 6,148,508 (Jeffrey A. Wolk) issued 11/21/00, 6,149,787 (Andrea W. Chow et al.) issued 11/21/00, 6,149,870 (J. Wallace Parce et al.) issued 11/21/00, 6,150,119 (Anne R. Kopf-Sill et al.) issued 11/21/00, 6,150,180 (J. Wallace Parce et al.) issued 11/21/00, 6,153,073 (Robert S. Dubrow et al.) issued 11/28/00, 6,156,181 (J. Wallace Parce et al.) issued 12/5/00, 6,167,910 (Calvin Y. H. Chow) issued 1/2/01, 6,171,067 (J. Wallace Parce) issued 1/9/01, 6,171,850 (Robert Nagle et al.) issued 1/9/01, 6,172,353 (Morten J. Jensen) issued 1/9/01, 6,174,675 (Calvin Y. H. Chow et al.) issued 1/16/01, 6,182,733 (Richard J. McReynolds) issued 2/6/01, 6,186,660 (Anne R. Kopf-Sill et al.) issued 2/13/01, 6,221,226 (Anne R. Kopf-Sill) issued April 24, 2001, 6,233,048 (J. Wallace Parce) issued May 15, 2001, 6,235,175 (Robert S. Dubrow et al.) issued 5/22/01, 6,235,471 (Michael Knapp et al.) issued 5/22/01, 6,238,538 (J. Wallace Parce et al.) issued 5/29/01, 6,251,343 (Robert S. Dubrow et al.) issued 6/26/01, 6,267,858 (J. Wallace Parce et al.) issued 7/31/01, 6,274,089 (Andrea W. Chow et al.) issued 8/14/01, 6,274,337 (J. Wallace Parce et al.) issued 8/14/01, 6,287,520 (J. Wallace Parce et al.) issued 9/11/01, 6,287,774 (Theo T. Nikiforov) issued 9/11/01, 6,303,343 (Anne R. Kopf-Sill) issued 10/16/01, 6,306,590 (Tammy Burd Mehta et al.) issued 10/23/01, and 6,306,659 (J. Wallace Parce et al.) issued 10/23/01.

Systems that are optionally adapted for use in the methods of the present invention are also described in, e.g., various published PCT applications, including WO 98/00231, WO 98/00705, WO 98/00707, WO 98/02728, WO 98/05424, WO 98/22811, WO 98/45481, WO 98/45929, WO 98/46438, and WO 98/49548, WO 98/55852, WO 98/56505, WO 98/56956, WO 99/00649, WO 99/10735, WO 99/12016, WO 99/16162, WO 99/19056, WO 99/19516, WO 99/29497, WO 99/31495, WO 99/34205, WO 99/43432, WO 99/44217, WO 99/56954, WO 99/64836, WO 99/64840, WO 99/64848, WO 99/67639, WO 00/07026, WO 00/09753, WO 00/10015, WO 00/21666, WO 00/22424, WO 00/26657, WO 00/42212, WO 00/43766, WO 00/45172, WO 00/46594, WO 00/50172, WO 00/50642, WO 00/58719, WO 00/60108, WO 00/70080, WO 00/70353, WO 00/72016, WO 00/73799, WO 00/78454, WO 01/02850, WO 01/14865, WO 01/17797, and WO 01/27253.

The methods of the invention are generally performed within fluidic channels. In some cases, as mentioned above, the channels are simply present in a capillary or pipettor element, e.g., a glass, fused silica, quartz, or plastic capillary. The capillary element is fluidly coupled to a source of, e.g., the reagent, sample, modulator, or other solution (e.g., by dipping the capillary element into a well on a microtiter plate), which is then flowed along the channel (e.g., a microchannel) of the element. The term "microfluidic," as used herein, generally refers to one or more fluid passages, chambers or conduits which have at least one internal cross-sectional dimension, e.g., depth, width, length, diameter, etc., that is less than 1 mm, and typically between about 0.1 µm and about 500 µm.

In the devices of the present invention, the microscale channels or cavities typically have at least one cross-sectional dimension between about 0.1 µm and 200 µm, preferably between about 0.1 µm and 100 µm, and often between about 0.1 µm and 50 µm. Accordingly, the microfluidic devices or systems prepared in accordance with the present invention typically include at least one microscale channel, usually at least two intersecting microscale channels, and often, three or more intersecting channels disposed within a single body structure that make up a channel network. Channel intersections may exist in a number of formats, including cross intersections, "Y" and/or "T" intersections, or any number of other structures whereby two channels are in fluid communication.

The body structures of the microfluidic devices described herein are typically manufactured from two or more separate portions or substrates which when appropriately mated or joined together, form the microfluidic device of the invention, e.g., containing the channels and/or chambers described herein. During body structure fabrication, the microfluidic devices described herein will typically include a top portion, a bottom portion, and an interior portion, wherein the interior portion substantially defines the covered channels and chambers of the device.

In one aspect, a bottom portion of the unfinished device includes a solid substrate that is substantially planar in structure, and which has at least one substantially flat upper surface. Channels are typically fabricated on one surface of the device and sealed (i.e., covered) by overlaying the channels with an upper substrate layer. A variety of substrate materials are optionally employed as the upper or bottom portion of the device. Typically, because the devices are microfabricated, substrate materials will be selected based upon their compatibility with known microfabrication techniques, e.g., photolithography, wet chemical etching, laser ablation, air abrasion techniques, LIGA, reactive ion etching, injection molding, embossing, and other techniques. The substrate materials are also generally selected for their compatibility with the full range of conditions to which the microfluidic devices may be exposed, including extremes of pH, temperature, electrolyte concentration, and/or for their chromatographic properties. Accordingly, in some preferred aspects, the substrate material may include materials normally associated with the semiconductor industry in which such microfabrication techniques are regularly employed, including, e.g., silica-based substrates, such as glass, quartz, silicon or polysilicon, as well as other substrate materials, such as gallium arsenide and the like. In the case of semiconductive materials, it will often be desirable to provide an insulating coating or layer, e.g., silicon oxide, over the substrate material, and particularly in those applications where electric fields are to be applied to the device or its contents.

In additional preferred aspects, the substrate materials will comprise polymeric materials, e.g., plastics, such as polymethylmethacrylate (PMMA), polycarbonate, polytetrafluoroethylene (TEFLON™), polyvinylchloride (PVC), polydimethylsiloxane (PDMS), polysulfone, polystyrene, polymethylpentene, polypropylene, polyethylene, polyvinylidine fluoride, ABS (acrylonitrile-butadiene-styrene copolymer), and the like. In preferred embodiments, at least the separation region(s) is/are fabricated from polyacrylamide, dimethylacrylamide, modified versions thereof, nonionic detergents, ionic detergents, or the like. Such polymeric substrates are readily manufactured using available microfabrication techniques, as described above, or from microfabricated masters, using known molding techniques, such as injection molding, embossing or stamping, or by polymerizing the polymeric precursor material within the mold (*see*, e.g., U.S. Patent No. 5,512,131). Such polymeric substrate materials are preferred for their ease of manufacture, low cost and disposability, as well as their general inertness to most extreme reaction conditions. Again, these polymeric materials optionally include treated surfaces, e.g., derivatized or coated surfaces, to enhance their utility in the microfluidic system, e.g., to provide enhanced fluid direction, e.g., as described in U.S. Pat. No. 5,885,470 (J. Wallace Parce et al.) issued 3/23/99.

The channels and/or cavities of the microfluidic devices are typically fabricated into the upper surface of the bottom substrate or portion of the device, as microscale grooves or indentations, using the above described microfabrication techniques. The top portion or substrate also comprises a first planar surface, and a second surface opposite the first planar surface. In the microfluidic devices prepared in accordance with certain aspects of the methods described herein, the top portion can include at least one aperture, hole, or port disposed therethrough, e.g., from the first planar surface to the second surface opposite the first planar surface. In other embodiments, the port(s) is/are optionally omitted, e.g., where fluids are introduced solely through external capillary elements.

The first planar surface of the top portion or substrate is then mated, e.g., placed into contact with, and bonded to the planar surface of the bottom substrate, covering and sealing the grooves and/or indentations in the surface of the bottom substrate, to form the channels and/or chambers (i.e., the interior portion) of the device at the interface of these two components. Bonding of substrates is typically carried out by any of a number of different methods, e.g., thermal bonding, solvent bonding, ultrasonic welding, and the like. The finished body structure of a device is a unitary structure that houses, e.g., the channels and/or chambers of the device.

The hole(s) in the top of the finished device is/are oriented to fluidly communicate with at least one of the channels and/or cavities. In the completed device, the hole(s) optionally function as reservoirs for facilitating fluid or material introduction into the channels or chambers of the device, as well as providing ports at which, e.g., pressure elements (e.g., vacuum sources, etc.) are optionally placed into contact with fluids within the device, allowing application of pressure gradients along the channels of the device to control and direct fluid transport within the device. In optional embodiments, extensions are provided over these reservoirs to allow for increased fluid volumes, permitting longer running assays, and better controlling fluid flow parameters, e.g., hydrostatic pressures. Examples of methods and apparatus for providing such extensions are described in U.S. Patent No. 6,251,343, filed February 24, 1998. These devices are optionally coupled to a sample introduction port, e.g., a pipettor or capillary element, which serially introduces multiple samples, e.g., from the wells of a microtiter plate. Thus, in some embodiments, both reservoirs in the upper surface and external capillary elements are present in a single device.

The sources of reagents, enzymes, substrates, samples, eluents, separation buffers, and other materials are optionally fluidly coupled to the microchannels in any of a variety of ways. In particular, those systems comprising sources of materials set forth in Knapp et al. "Closed Loop Biochemical Analyzers" (WO 98/45481; PCT/US98/06723) and U.S. Pat. No. 5,942,443 issued August 24, 1999, entitled "High Throughput Screening Assay Systems in Microscale Fluidic Devices" to J. Wallace Parce et al. and, e.g., in 60/128,643 filed April 4, 1999, entitled "Manipulation of Microparticles In Microfluidic Systems," by Mehta et al. are applicable.

In these systems and as noted above, a capillary or pipettor element (i.e., an element in which components are optionally moved from a source to a microscale element such as a second channel or reservoir) is temporarily or permanently coupled to a source of material. The source is optionally internal or external to a microfluidic device that includes the pipettor or capillary element. Example sources include microwell plates, membranes or other solid substrates comprising lyophilized components, wells or reservoirs in the body of the microscale device itself and others.

### FLOW OF MATERIALS IN MICROFLUIDIC SYSTEMS

A preferred method of flowing materials along the microchannels or other cavities of the devices described herein is by pressure-based flow. Pressure is applied with or without a simultaneously applied electric field. Application of a pressure differential along a channel is carried out by any of a number of approaches. For example, it may be desirable to provide relatively precise control of the flow rate of materials, e.g., to precisely control incubation or separation times, etc. As such, in many preferred aspects, flow systems that are more active than hydrostatic pressure driven systems are employed. In certain cases, reagents may be flowed by applying a pressure differential across the length of the analysis channel. For example, a pressure source (positive or negative) is applied at the reagent reservoir at one end of the analysis channel, and the applied pressure forces the reagents/samples through the channel. The pressure source is optionally pneumatic, e.g., a pressurized gas, or a positive displacement mechanism, i.e., a plunger fitted into a reagent reservoir, for forcing the reagents through the analysis channel. Alternatively, a vacuum source is applied to a reservoir at the opposite end of the channel to draw the reagents through the channel. *See,* FIGS. 1 and 5. Pressure or vacuum sources may be supplied external to the device or system, e.g., external vacuum or pressure pumps sealably fitted to the inlet or outlet of the analysis channel, or they may be internal to the device, e.g., microfabricated pumps integrated into the device and operably linked to the analysis channel. Examples of microfabricated pumps have been widely described in the art. *See,* e.g., published International Application No. WO 97/02357.

In an alternative simple passive aspect, the reagents are deposited in a reservoir or well at one end of an analysis channel and at a sufficient volume or depth, that the reagent sample creates a hydrostatic pressure differential along the length of the analysis channel, e.g., by virtue of it having greater depth than a reservoir at an opposite terminus of the channel. The hydrostatic pressure then causes the reagents to flow along the length of the channel. Typically, the reservoir volume is quite large in comparison to the volume or flow through rate of the channel, e.g., 10 µl reservoirs, vs. 1000 µm² channel cross-section. As such, over the time course of the assay/separation, the flow rate of the reagents will remain substantially constant, as the volume of the reservoir, and thus, the hydrostatic pressure changes very slowly. Applied pressure is then readily varied to yield different reagent flow rates through the channel. In screening applications, varying the flow rate of the reagents is optionally used to vary the incubation time of the reagents. In particular, by slowing the flow rate along the channel, one can effectively lengthen the amount of time between introduction of reagents and detection of a particular effect. Alternatively, analysis channel lengths, detection points, or reagent introduction points are varied in fabrication of the devices, to vary incubation times. *See also*, "Multiport Pressure Control System," by Chien and Parce, USSN 60/184,390, filed February 23, 2000, which describes multiport pressure controllers that couple pumps to multiple device reservoirs.

In further alternate aspects, hydrostatic, wicking and capillary forces are additionally, or alternately, used to provide for fluid flow. *See,* e.g., "Method and Apparatus for Continuous Liquid Flow in Microscale Channels Using Pressure Injection, Wicking and Electrokinetic Injection," by Alajoki et al., USSN 09/245,627, filed February 5, 1999. In these methods, an adsorbent material or branched capillary structure is placed in fluidic contact with a region where pressure is applied, thereby causing fluid to move towards the adsorbent material or branched capillary structure.

In alternative aspects, flow of reagents is driven by inertial forces. In particular, the analysis channel is optionally disposed in a substrate that has the conformation of a rotor, with the analysis channel extending radially outward from the center of the rotor. The reagents are deposited in a reservoir that is located at the interior portion of the rotor and is fluidly connected to the channel. During rotation of the rotor, the centripetal force on the reagents forces the reagents through the analysis channel, outward toward the edge of the rotor. Multiple analysis channels are optionally provided in the rotor to perform multiple different analyses. Detection of a detectable signal produced by the reagents is then carried out by placing a detector under the spinning rotor and detecting the signal as the analysis channel passes over the detector. Examples of rotor systems have been previously described for performing a number of different assay types. *See,* e.g., Published International Application No. WO 95/02189. Test compound reservoirs are optionally provided in the rotor, in fluid communication with the analysis channel, such that the rotation of the rotor also forces the test compounds into the analysis channel.

For purposes of illustration, the discussion has focused on a single channel and accessing capillary; however, it will be readily appreciated that these aspects may be provided as multiple parallel analysis channels (e.g., each including mixing and separation regions) and accessing capillaries, in order to substantially increase the throughput of the system. Specifically, single body structures may be provided with multiple parallel analysis channels coupled to multiple sample accessing capillaries that are positioned to sample multiple samples at a time from sample libraries, e.g., multiwell plates. As such, these capillaries are generally spaced at regular distances that correspond with the spacing of wells in multiwell plates, e.g., 9 mm centers for 96 well plates, 4.5 mm for 384 well plates, and 2.25 mm for 1536 well plates.

In alternate aspects, an applied pressure is accompanied by the simultaneous application of an electric field to further effect fluid transport, e.g., through the mixing and/or separation regions of the microchannel. The electrokinetic transport systems of the invention typically utilize electric fields applied along the length of microchannels that have a surface potential or charge associated therewith. When fluid is introduced into the microchannel, the charged groups on the inner surface of the microchannel ionize, creating locally concentrated levels of ions near the fluid surface interface. Under an electric field, this charged sheath migrates toward the cathode or anode (depending upon whether the sheath comprises positive or negative ions) and pulls the encompassed fluid along with it, resulting in bulk fluid flow. This flow of fluid is generally termed electroosmotic flow. Where the fluid includes reagents (e.g., materials to be separated), the reagents are also pulled along. A more detailed description of controlled electrokinetic material transport systems in microfluidic systems is described in published International Patent Application No. WO 96/04547.

### INTEGRATED SYSTEMS

The present invention, in addition to other integrated system components, also optionally includes a microfluidic device handler for performing the methods disclosed herein. Specifically, the microfluidic device handler includes a holder configured to receive the microfluidic device, a container sampling region proximal to the holder, and the controller. During operation of the handler, the controller directs, e.g., dipping of microfluidic device capillary or pipettor element(s) into a portion of, e.g., a microwell plate in the container sampling region. The microfluidic device handler also optionally includes a computer or a computer readable medium operably connected to the controller. The computer or the computer readable medium typically includes an instruction set for varying or selecting a rate or a mode of dipping capillary element(s) into fluid materials.

Although the devices and systems specifically illustrated herein are generally described in terms of the performance of a few or one particular operation, it will be readily appreciated from this disclosure that the flexibility of these systems permits easy integration of additional operations into these devices. For example, the devices and systems described will optionally include structures, reagents and systems for performing virtually any number of operations in addition to the operations specifically described herein. Aside from fluid handling, assays, and separation of sample and/or reaction components, other upstream or downstream operations include, e.g., extraction, purification, amplification, cellular activation, labeling reactions, dilution, aliquotting, labeling of components, assays and detection operations, electrokinetic or pressure-based injection of components or materials into contact with one another, or the like. Assay and detection operations include, without limitation, cell fluorescence assays, cell activity assays, receptor/ligand assays, immunoassays, or the like.

In the present invention, the separated materials are optionally monitored and/or detected so that, e.g., an activity can be determined. The systems described herein generally include microfluidic device handlers, as described above, in conjunction with additional instrumentation for controlling fluid transport, flow rate and direction within the devices, detection instrumentation for detecting or sensing results of the operations performed by the system, processors, e.g., computers, for instructing the controlling instrumentation in accordance with preprogrammed instructions, receiving data from the detection instrumentation, and for analyzing, storing and interpreting the data, and providing the data and interpretations in a readily accessible reporting format.

### Controllers

A controller **130** of system **100** of the present invention directs dipping of capillary elements into, for example, multiwell plate **150** to sample reagents such as enzymes and substrates, fluid recirculation baths or troughs, or the like. A variety of controlling instrumentation is also optionally utilized in conjunction with the microfluidic devices and handling systems described herein, for controlling the transport, concentration, direction, and motion of fluids and/or separation of materials within the devices of the present invention, e.g., by pressure-based control.

As described above, in many cases, fluid transport, concentration, and direction are controlled in whole or in part, using pressure based flow systems that incorporate external or internal pressure sources to drive fluid flow. Internal sources include microfabricated pumps, e.g., diaphragm pumps, thermal pumps, and the like that have been described in the art. *See,* e.g., U.S. Patent Nos. 5,271,724, 5,277,556, and 5,375,979 and Published PCT Application Nos. WO 94/05414 and WO 97/02357. Preferably, external pressure sources are used, and applied to ports at channel termini. These applied pressures, or vacuums, generate pressure differentials across the lengths of channels to drive fluid flow through them. In the interconnected channel networks described herein, differential flow rates on volumes are optionally accomplished by applying different pressures or vacuums at multiple ports, or preferably, by applying a single vacuum at a common waste port (*see*, Figure 1) and configuring the various channels with appropriate resistance to yield desired flow rates. Example systems are also described in USSN 09/238,467 filed 1/28/99.

Typically, the controller systems are appropriately configured to receive or interface with a microfluidic device or system element as described herein. For example, the controller and/or detector, optionally includes a stage upon which the device of the invention is mounted to facilitate appropriate interfacing between the controller and/or detector and the device. Typically, the stage includes an appropriate mounting/alignment structural element, such as a nesting well, alignment pins and/or holes, asymmetric edge structures (to facilitate proper device alignment), and the like. Many such configurations are described in the references cited herein.

The controlling instrumentation discussed above is also used to provide for electrokinetic injection or withdrawal of material downstream of the region of interest to control an upstream flow rate. The same instrumentation and techniques described above are also utilized to inject a fluid into a downstream port to function as a flow control element.

### Detector

The devices described herein include signal detectors **122** which detect fluorescence. In other devices, signal detectors may detect concentration, phosphorescence, radioactivity, pH, charge, absorbance, refractive index, luminescence, temperature, magnetism, mass (e.g., mass spectrometry), or the like. The detector(s) optionally monitors one or a plurality of signals from upstream and/or downstream (e.g., in or proximal to the separation region) of an assay mixing point in which, e.g., a ligand and an enzyme are mixed. For example, the detector optionally monitors a plurality of optical signals which correspond in position to "real time" assay/separation results.

Example detectors or sensors include photomultiplier tubes, CCD arrays, optical sensors, temperature sensors, pressure sensors, pH sensors, conductivity sensors, mass sensors, scanning detectors, or the like. Materials which emit a detectable signal are optionally flowed past the detector, or, alternatively, the detector can move relative to the array to determine the position of an assay component (or, the detector can simultaneously monitor a number of spatial positions corresponding to channel regions, e.g., as in a CCD array). Each of these types of sensors is optionally readily incorporated into the microfluidic systems described herein. In these systems, such detectors are placed either within or adjacent to the microfluidic device or one or more channels, chambers or conduits of the device, such that the detector is within sensory communication with the device, channel, or chamber. The phrase "within sensory communication" of a particular region or element, as used herein, generally refers to the placement of the detector in a position such that the detector is capable of detecting the property of the microfluidic device, a portion of the microfluidic device, or the contents of a portion of the microfluidic device, for which that detector was intended. The detector optionally includes or is operably linked to a computer, e.g., which has software for converting detector signal information into assay result information (e.g., kinetic data of modulator activity), or the like. A microfluidic system optionally employs multiple different detection systems for monitoring the output of the system. Detection systems of the present invention are used to detect and monitor the materials in a particular channel region (or other reaction detection region).

The detector optionally exists as a separate unit, but is preferably integrated with the controller system, into a single instrument. Integration of these functions into a single unit facilitates connection of these instruments with the computer (described below), by permitting the use of few or a single communication port(s) for transmitting information between the controller, the detector, and the computer.

### Computer

As noted above, the microfluidic devices and integrated systems of the present invention include a computer **120** operably connected to the controller. The computer typically includes an instruction set, e.g., for varying or selecting a rate or a mode of dipping capillary or pipettor elements into fluid materials, for sampling fluidic materials (e.g., enzymes, substrates, reactants, chromatographic materials, eluents, separation buffers, etc.), or the like. Additionally, either or both of the controller system and/or the detection system is/are optionally coupled to an appropriately programmed processor or computer which functions to instruct the operation of these instruments in accordance with preprogrammed or user input instructions, receive data and information from these instruments, and interpret, manipulate and report this information to the user. As such, the computer is typically appropriately coupled to one or both of these instruments (e.g., including an analog to digital or digital to analog converter as needed).

The computer typically includes appropriate software for receiving user instructions, either in the form of user input into a set parameter fields, e.g., in a GUI, or in the form of preprogrammed instructions, e.g., preprogrammed for a variety of different specific operations. The software then converts these instructions to appropriate language for instructing the operation of the fluid direction and transport controller to carry out the desired operation, e.g., varying or selecting the rate or mode of fluid and/or microfluidic device movement, controlling flow rates within microscale channels, directing xyz translation of the microfluidic device or of one or more microwell plates, or the like. The computer then receives the data from the one or more sensors/detectors included within the system, and interprets the data, either provides it in a user understood format, or uses that data to initiate further controller instructions, in accordance with the programming, e.g., such as in monitoring and control of flow rates, temperatures, applied voltages, and the like. Additionally, the software is optionally used to control, e.g., pressure or electrokinetic modulated injection or withdrawal of material.

### EXAMPLE 2:

In vitro screening of kinase activity typically involves the use of a short peptide sequence used as an in vitro surrogate for the physiologically relevant protein substrate. An alternative embodiment of the invention may be employed to identify such substrate surrogates. Substrate plates for this purpose are arranged based on considerations similar to those taken into account for substrate plates that are matched with an enzyme plate as described above. I.e., the substrates are arranged based on factors such as, for example, electrophoretic mobility.

In an example assay, a kinase target of interest is incubated in a micro titer plate with a library of fluorescently labeled peptide substrate-candidates. The reaction is terminated and the mixture sipped onto a microfluidic chip. The extent of phosphorylation of each fluorescently labeled peptide is then measured by a mobility shift assay in accordance with the invention.

A control solution that contains only the peptide without any enzyme is provided for each peptide substrate-candidate. Thus, each multiwell plate includes two solutions containing each peptide, one solution without enzyme and one solution with enzyme. The control solution contains peptide, ATP, and reaction buffer. The enzyme solution contains the same components plus the enzyme. Both the control and enzyme-containing solutions are incubated for a predetermined period of time. At the end of that time, the reaction in the well with the enzyme-containing solution is terminated by the addition of an amount of termination buffer, while the same amount of separation buffer is added to the well containing the control solution. Addition of the same amount of material to both wells ensures that the final concentration of the labeled peptide in both wells is identical.

This example assay is carried out in a microfluidic device containing multiple external capillaries or "sippers" that allow samples from multiple sources to be simultaneously introduced to be processed in parallel within the microfluidic device. Examples of such microfluidic devices are provided in U.S. Patent No. 6,358,387, which is assigned to the assignee of this invention. The location of the control well on the multiwell plate is such that while the solution containing both the enzyme and a particular substrate candidate is sipped on one sipper of a sipper chip, the control solution containing that same substrate candidate is also sipped onto the same chip through another sipper. After the enzyme-containing and control solutions enter the microfluidic device, both solutions are subjected to a common set of separation conditions (i.e. pressure and voltages). The use of the control solution becomes most critical when there is a high level of substrate conversion in the reaction well, as only one peak is present and so comparison of arrival time of peaks in the channels containing the enzyme-containing and control solutions indicates the presence of product formation.

In general, to achieve a desired resolution during the separation of a given peptide substrate and the product resulting from the interaction between the substrate and an enzyme, an optimum set of pressures and separation voltages are used. This would mean that for a panel of 54 peptides, 54 unique separation conditions would be used. Even with the automation afforded by the use of a microfluidic device, it may be cumbersome to manually input the separation conditions required for each peptide. A compromise solution involves grouping peptides that have similar separation conditions and separating them under a common set of conditions. For example, a constant pressure could be applied during the assay of each peptide, while different voltages could be applied for each different group of peptides.

In many embodiments, the substrate-candidate peptides can be broadly grouped into two groups: product-first and substrate-first assays. In a product-first assay, the product peak of a product-substrate pair reaches the detector first. An operating pressure was designated for product- and substrate-first assays; in our specific case we chose -1 psi for product-first assays and -1.6 for substrate-first assays. Next, voltage drops were chosen that give a resolution of 1.2 at the operating pressure. Peptides were then grouped together into groups of 6 where the operating voltage drops were within 200V of each other. Peptides were grouped into groups of 6 because a 12-sipper chip could sip 6 enzyme-containing wells and 6 control wells simultaneously. (Note: if a 4 sipper chip were used, peptides could be grouped into groups of 2.) An operating voltage drop was then chosen for this group of 6 peptides by choosing highest voltage drop common to all 6 peptides; in this way each product-substrate pair had a resolution of 1.2 or greater and assay sensitivity was not compromised. In some cases a group of 6 peptides could not be made and so a group of fewer than six was made with the balance being buffer-only wells. So in the case of 54 peptides used in our library, 21 were chosen to be product-first, which translated into 4 groups of 6 peptides with 3 buffer only wells. All groups operated at the same pressure of -1 psi, but each group had an incrementally increasing voltage drop as designated within a multi-line voltage script. The timing of application of the successive voltage drops was determined such that the peptides from the previous group had passed the detection zone and the new peptides had not yet been sipped. This in turn, coupled with the separation conditions, determined the throughput of the assay. The remaining 33 peptides were grouped on a separate plate and run substrate-first at a common pressure of -1.6 psi, resulting in 6 groups of 6 peptides with 3 buffer-only wells. Choice of voltage and timing of application of voltage is identical to the product-first case.

The substrates are placed on a multiwell plate in DMSO in a concentrated format such that when the enzyme and reaction buffer is used, the final substrate concentration is close to the desired assay conditions, e.g., store 1 µl of 76 gM substrate and then add to 50 p1 of reaction buffer for a final concentration of 1.5 µM. These plates can then be made in bulk and frozen for use at a later date.

Without knowledge of the enzymatic kinetics, the reactions are typically carried out with a multi-hour incubation (> 12 hours) and with high enzyme and ATP concentrations.

With this assay format, 12 kinase enzymes were run to look for substrates. One enzyme was PICA and was used only as a test case to see if the method found our regular kinase target.

Of the other 11 kinases, we found new targets for 8 of them. These enzymes and their new substrates are listed in the following table.

| **Enzyme** | **[E] (nM)** | **[ATP] (µM)** | **Co-Factor** | **Literature Substrate (% Conversion)** | **Hits** |
|---|---|---|---|---|---|
| MET | 10 | 250 | MnCl₂ | KKKSPGEYVNIEFG (70%) | FL-EDPIYEFLPAKKK-CONH2 (97%) |
| | | | | | FL-MAEEEIYGEFFAKKK-CONH2 (93%) |
| | | | | | FL-KMAEEEEYFELVAKKK-CONH2 (90%) |
| | | | | | FL-EAIYAAPFAKKK-CONH2 (88%) |
| | | | | | FL-EEEEYFFIIAKKK-CONH2 (86%) |
| | | | | | FL-KMAEEEEYVFIEAKKK-CONH2 (85%) |
| | | | | | FL-KEDPDYEWPSAK -CONH2(63%) |
| | | | | | FL-MAAEEEYFFLFAKKK-CONH2 (62%) |
| | | | | | FL-EGIYGVLFKKK-CONH2 (60%) |
| MST2 | 10 | 100 | MnCl₂ | MBP (N/A) | FL-KKSRGDYMTMQIG-CONH2 (75%) |
| | | | | | FL-ENDYINASLKKK-CONH2 (60%) |
| | | | | | FL-PLARTLSVAGLPGKK-COOH(40%) |
| IKKα | 3.5 | 100 | MnCl₂ | IKK-tide (N/A) | FL-AKRRRLSSLRA-COOH (43%) |
| IGF-1R | 10 | 250 | MnCl₂ | KKKSPGEYVNIEFG (30%) | FL-KKSRGDYMTMQIG-CONH2 (55%) |
| | | | | | FL-MAAEEEYFFLFAKKK-CONH2 (60%) |
| FGFR3 | 5 | 250 | MnCl₂ | Poly-Glu Tyr (N/A) | FL-EDPIYEFLPAKKK-CONH2 (100%) |
| | | | | | FL-KMAEEEEYFELVAKKK-CONH2 (100%) |
| | | | | | FL-KMAEEEEYFELVAKKK-CONH2 (100%) |
| | | | | | FL-KMAEEEEYVFIEAKKK-CONH2 (100%) |
| | | | | | FL-MAAEEEYMMMMAKKK-CONH2 (78%) |
| | | | | | FL-KKKSPGEYVNIEFG-CONH2 (73%) |
| EGFR | 5 | 250 | MnCl₂ | Angiotensin II (N/A) | FL-EDPIYEFLPAKKK-CONH2 (98%) |
| | | | | | FL-KMAEEEEYFEL VAKKK-CONH2(90%) |
| | | | | | FL-MAEEEIYGEFFAKKK-CONH2 (87%) |
| | | | | | FL-KKKSPGEYVNIEFG-CONH2 (50%) |
| | | | | | FL-KEDPDYEWPSAK-CONH2 (50%) |
| | | | | | FL-EAIYAAPFAKKK-CONH2 (46%) |
| | | | | | FL-ENDYINASLKKK-CONH2(44%) |
| IKKβ | | | MnCl₂ | IKK-tide (N/A) | FL-AKRRRLSSLRA-COOH (74%) |
| ERK2 (MAPK2) | | | | | FL-IPTSPITTT YIFFKKK-COOH (100%) |
| | | | | | FL-APRTPGGRR-COOH (60%) |
| MEK1 | 7 | 100 | MnCl₂ | N/A | No Hit |
| JNK2a | 60 | 100 | MnCl₂ | N/A | No Hit |
| MKK6 | | | | | No Hit |

This table lists the substrates as hits with the peptide sequence and the 90 conversion in parentheses. Where possible, data is shown for the enzyme with a known peptide sequence for the enzyme. In addition, assay conditions are listed. In all cases, the enzyme and substrate were incubated overnight (i.e., ∼ 12-16 hours).

While the foregoing invention has been described in some detail for purposes of clarity and understanding, it will be clear from a reading of this disclosure that various changes in form and detail can be made without departing from the true scope of the invention, as defined by the claims. For example, all the techniques and apparatus described above may be used in various combinations.

## Claims

1. A kit for screening a compound for enzyme inhibition activity, the kit comprising:
a first multiwell plate **150** having a plurality of enzymes disposed within a first plurality of wells;
a second multiwell plate **160** having a plurality of enzyme substrates disposed within a second plurality of wells, wherein the order in which the enzymes are disposed on the first plate is determined by the order in which the corresponding enzyme substrates are disposed on the second plate;
a phosphate source **162** disposed within each well of the second plate, the phosphate source disposed in the well at a concentration specific for the enzyme disposed in the corresponding well of the first plate; and
a cofactor **164** disposed within each well of the second plate, the cofactor disposed in each well at a concentration specific for the enzyme disposed in the corresponding well of the first plate, **characterised in that** the first plurality of wells of the first multiwell plate are disposed evenly upon the plate in 24 columns **250,** each column including a different enzyme disposed within the wells of the column wherein:
(i) the order in which the enzymes are disposed on the first plate from column 1 to column 24 is MAPKAPK2, AurA, PKCζ, RSK1, MAPKAPK5, Erk1, PKD2, CK1δ, CHK1, ABL, FYN, LYNa, CHK2, MET, LCK, SRC, GSK3β, Erk2, PKACα, AKT2, INSR, p38α, AKT1, and MSK1; or
(ii) the order in which the enzymes are disposed on the first plate from column 1 to column 24 is PKCβ2, ROCK2, CDK2, MST2, PKG1α, PAK2, IGF1R, FGFR1, MARK1, CAMK2δ, PIM2, BTK, c-TAK1, DYRKIa, CaMK4, FLT3, HGK, KDR, Raf-1, P70S6K, IRAK4, SGK, and SYK.

2. The kit of claim 1 further comprising a reconstitution buffer **152** and/or a termination buffer **158.**

3. A system for screening a compound for enzyme inhibition activity, the system comprising:
a kit according to claim 1 or claim 2; **characterised in that** the system further comprises:
a microfluidic device **110,** the microfluidic device having at least one microchannel **102** to which aliquots of sample from each well of the first plate **150** are provided for separation and analysis, and a capillary element **112,** the capillary element operably connected to and in fluid communication with the microchannel; and
microfluidic device handlers to control the translation of the microfluidic device **110** relative to the first plate **150** under the direction of a computer **120** and transfer of materials from the second plate **160** to the first plate **150;**

4. The system of claim 3 further comprising:
(i) a detector **122** operably connected to the microfluidic device and a computer **120;** and/or
(ii) a controller **130** operably connected to the computer **120** and optionally further comprising a fluid direction system **140** operably connected to the computer **120,** the fluid direction system including a pressure source **142** in fluid communication with the microfluidic device.

5. A method of screening a compound for enzyme inhibition activity, the method comprising:
preparing at least one sample mixture using a kit according to claim 1 or claim 2;
providing the at least one sample mixture to a microfluidic device, the sample mixture comprising an enzyme, an enzyme substrate, a test compound, and a product;
applying vacuum pressure to the sample mixture;
flowing the sample mixture along a microchannel of the microfluidic device in response to the applied pressure;
separating the product and the enzyme substrate based upon a difference in electrophoretic mobility between the product and the enzyme substrate;
detecting the separated product and enzyme substrate, and
determining enzyme inhibition activity of the compound based on the detection of the separated product and enzyme substrate.

6. The method of claim 5 wherein providing the at least one sample mixture to the microfluidic device comprises flowing the sample mixture from a well of the first plurality of wells into the microchannel via a capillary element, the capillary element operably connected to and in fluid communication with the microchannel.

7. The method of claim 5 wherein the at least one sample mixture is selected from the first plurality of wells, the first multiwell plate having a plurality of columns of wells, wherein each well of each column includes a single enzyme.

8. The method of claim 5 wherein preparing the at least one sample mixture using the kit according to claim 1 or 2 comprises: thawing the first and second multiwell plates, the first and second multiwell plates having been stored frozen; adding a reconstitution buffer to each well of the first plurality of wells; adding a compound to each well of the first plurality of wells; adding an enzyme substrate from each of the second plurality of wells to each corresponding well of the first plurality of wells; and adding a termination buffer to each of the first plurality of wells.

## Patentansprüche

1. Set zum Screenen einer Verbindung auf Enzymhemmungsaktivität, wobei das Set Folgendes umfasst:
eine erste Multiwell-Platte **150** mit einer Vielzahl von Enzymen, die in einer ersten Vielzahl von Wells angeordnet sind;
eine zweite Multiwell-Platte **160** mit einer Vielzahl von Enzymsubstraten, die in einer zweiten Vielzahl von Wells angeordnet sind, wobei die Reihenfolge, in der die Enzyme auf der ersten Platte angeordnet sind, von der Reihenfolge bestimmt wird, in der die entsprechenden Enzymsubstrate auf der zweiten Platte angeordnet sind;
eine Phosphatquelle **162,** die in jedem Well der zweiten Platte angeordnet ist, wobei die Phosphatquelle im Well in einer Konzentration angeordnet ist, die für das Enzym, das im entsprechenden Well der ersten Platte angeordnet ist, spezifisch ist; und
einen Cofaktor **164,** der in jedem Well der zweiten Platte angeordnet ist, wobei der Cofaktor in jedem Well in einer Konzentration angeordnet ist, die für das Enzym, das im entsprechenden Well der ersten Platte angeordnet ist, spezifisch ist, **dadurch gekennzeichnet, dass** die erste Vielzahl von Wells der ersten Multiwell-Platte auf der Platte gleichmäßig in 24 Spalten **250** angeordnet ist, wobei jede Spalte ein anderes, in den Wells der Spalte angeordnetes Enzym umfasst, wobei:
(i) die Reihenfolge, in der die Enzyme auf der ersten Platte von Spalte 1 bis Spalte 24 angeordnet sind, MAPKAPK2, AurA, PKCζ, RSK1, MAPKAPK5, Erk1, PKD2, CK1δ, CHK1, ABL, FYN, LYNa, CHK2, MET, LCK, SRC, GSK3β, Erk2, PKACα, AKT2, INSR, p38a, AKT1 und MSK1 ist; oder
(ii) die Reihenfolge, in der die Enzyme auf der ersten Platte von Spalte 1 bis Spalte 24 angeordnet sind, PKCβ2, ROCK2, CDK2, MST2, PKG1α, PAK2, IGF1R, FGFR1, MARK1, CAMK2δ, PIM2, BTK, c-TAK1, DYRKIa, CaMK4, AMPKα1, FLT3, HGK, KDR, Raf-1, P70S6K, IRAK4, SGK und SYK ist.

2. Set nach Anspruch 1, das weiters einen Rekonstitutionspuffer 152 und/oder einen Terminationspuffer **158** umfasst.

3. System zum Screenen einer Verbindung auf Enzymhemmungsaktivität, wobei das System Folgendes umfasst:
ein Set nach Anspruch 1 oder 2; **dadurch gekennzeichnet, dass** das System weiters Folgendes umfasst:
eine Mikrofluidikvorrichtung **110**, wobei die Mikrofluidikvorrichtung zumindest einen Mikrokanal **102**, für den von jedem Well der ersten Platte **150** Probenaliquote zur Trennung und Analyse bereitgestellt werden, und ein Kapillarelement **112** aufweist, wobei das Kapillarelement mit dem Mikrokanal operabel verbunden ist und mit diesem in Fluidkommunikation steht; und
Mikrofluidikvorrichtungssteuerungen, um die Verschiebung der Mikrofluidikvorrichtung **110** relativ zur ersten Platte **150** unter der Steuerung eines Computers **120** und die Übertragung von Materialien von der zweiten Platte **160** zur ersten Platte **150** zu steuern.

4. System nach Anspruch 3, das weiters Folgendes umfasst:
(i) einen Detektor **122**, der mit der Mikrofluidikvorrichtung operabel verbunden ist, und einen Computer **120**; und/oder
(ii) einen Controller **130,** der mit dem Computer **120** operabel verbunden ist und gegebenenfalls außerdem ein Fluidlenkungssystem **140** umfasst, das mit dem Computer **120** operabel verbunden ist, wobei das Fluidlenkungssystem eine Druckquelle **142** umfasst, die in Fluidkommunikation mit der Mikrofluidikvorrichtung steht.

5. Verfahren zum Screenen einer Verbindung auf Enzymhemmungsaktivität, wobei das Verfahren Folgendes umfasst:
das Herstellen von zumindest einem Probengemisch unter Verwendung eines Sets nach Anspruch 1 oder Anspruch 2;
das Bereitstellen des zumindest einen Probengemischs für eine Mikrofluidikvorrichtung, wobei das Probengemisch ein Enzym, ein Enzymsubstrat, eine Testverbindung und ein Produkt umfasst;
das Ausüben von Vakuumdruck auf das Probengemisch;
das Strömenlassen des Probengemischs durch einen Mikrokanal der Mikrofluidikvorrichtung als Reaktion auf den ausgeübten Druck;
das Trennen des Produkts und des Enzymsubstrats auf Basis eines Unterschieds in der elektrophoretischen Mobilität zwischen dem Produkt und dem Enzymsubstrat;
das Detektieren des getrennten Produkts und Enzymsubstrats; und
das Bestimmen einer Enzymhemmungsaktivität der Verbindung auf Basis der Detektion des getrennten Produkts und Enzymsubstrats.

6. Verfahren nach Anspruch 5, wobei das Bereitstellen des zumindest einen Probengemischs für die Mikrofluidikvorrichtung das Strömenlassen des Probengemischs aus einem Well der ersten Vielzahl von Wells über ein Kapillarelement in den Mikrokanal umfasst, wobei das Kapillarelement mit dem Mikrokanal operabel verbunden ist und mit diesem in Fluidkommunikation steht.

7. Verfahren nach Anspruch 5, wobei das zumindest eine Probengemisch aus der ersten Vielzahl von Wells ausgewählt ist, wobei die erste Multiwell-Platte eine Vielzahl von Well-Spalten aufweist, wobei jedes Well aus jeder Spalte ein einziges Enzym umfasst.

8. Verfahren nach Anspruch 5, wobei das Herstellen des zumindest einen Probengemischs unter Verwendung eines Sets nach Anspruch 1 oder 2 Folgendes umfasst: das Auftauen der ersten und der zweiten Multiwell-Platte, nachdem die erste und die zweite Multiwell-Platte gefroren gelagert wurden; das Zusetzen eines Rekonstitutionspuffers zu jedem Well aus der ersten Vielzahl von Wells; das Zusetzen einer Verbindung zu jedem Well aus der ersten Vielzahl von Wells; das Zusetzen eines Enzymsubstrats aus jedem aus der zweiten Vielzahl von Wells zu jedem entsprechenden Well der ersten Vielzahl von Wells; und das Zusetzen eines Terminationspuffers zu jedem aus der ersten Vielzahl von Wells.

## Revendications

1. Kit pour le criblage d'un composé en vue d'une activité d'inhibition enzymatique, le kit comprenant :
une première plaque multipuits 150 ayant une pluralité d'enzymes disposées dans une première pluralité de puits ;
une seconde plaque multipuits 160 ayant une pluralité de substrats enzymatiques disposés dans une seconde pluralité de puits, dans lequel l'ordre dans lequel les enzymes sont disposées sur la première plaque est déterminé par l'ordre dans lequel les substrats enzymatiques correspondants sont disposés sur la seconde plaque ;
une source de phosphate 162 disposée dans chaque puits de la seconde plaque, la source de phosphate étant disposée dans le puits à une concentration spécifique pour l'enzyme disposée dans le puits correspondant de la première plaque ; et
un cofacteur 164 disposé dans chaque puits de la seconde plaque, le cofacteur disposé dans chaque puits à une concentration spécifique pour l'enzyme disposée dans le puits correspondant de la première plaque, **caractérisé en ce que** la première pluralité de puits de la première plaque multipuits sont disposés uniformément sur la plaque dans 24 colonnes 250, chaque colonne comprenant une enzyme différente disposée dans les puits de la colonne, dans lequel :
(i) l'ordre dans lequel les enzymes sont disposées sur la première plaque de la colonne 1 à la colonne 24 est MAPKAPK2, AurA, PKCζ, RSK1, MAPKAPKS, Erkl, PKD2, CK1δ, CHK1, ABL, FYN, LYNa, CHK2, MET, LCK, SRC, GSK3β, Erk2, PKACα, AKT2, INSR, p38a, AKT1 et MSK1 ; ou
(ii) l'ordre dans lequel les enzymes sont disposées sur la première plaque de la colonne 1 à la colonne 24 est PKOPIβ2 ROCK2, CDK2, MST2, PKGlα, PAK2, IGF1R, FGFR1, MARK1, CAMK2δ, PIM2, BTK, c-TAK1, DYRKIa, CaMK4, AMPKαl, FLT3, HGK, KDR, Raf-1, P70S6K, IRAK4, SGK et SYK.

2. Kit selon la revendication 1, comprenant en outre un tampon de reconstitution 152 et/ou un tampon de terminaison 158.

3. Système de criblage d'un composé pour l'activité d'inhibition enzymatique, le système comprenant :
un kit selon la revendication 1 ou la revendication 2 ;
**caractérisé en ce que** le système comprend en outre :
un dispositif microfluidique 110, le dispositif microfluidique ayant au moins un microcanal 102 auquel des aliquotes d'échantillon provenant de chaque puits de la première plaque 150 sont fournis pour séparation et analyse, et un élément capillaire 112, l'élément capillaire connecté de manière fonctionnelle et en communication fluidique avec le microcanal ; et
des dispositifs de manipulation de dispositif microfluidique pour commander la translation du dispositif microfluidique 110 par rapport à la première plaque 150 sous la direction d'un ordinateur 120 et un transfert de matériaux de la seconde plaque 160 à la première plaque 150 ;

4. Système selon la revendication 3, comprenant en outre :
(i) un détecteur 122 connecté de manière opérationnelle au dispositif microfluidique et à un ordinateur 120 ; et/ou
(ii) un dispositif de commande 130 connecté de manière opérationnelle à l'ordinateur 120 et comprenant facultativement en outre un système de direction de fluide 140 connecté de manière opérationnelle à l'ordinateur 120, le système de direction de fluide comprenant une source de pression 142 en communication de fluide avec le dispositif microfluidique.

5. Procédé de criblage d'un composé pour l'activité d'inhibition enzymatique, le procédé comprenant les étapes consistant à :
préparer au moins un mélange échantillon en utilisant un kit selon la revendication 1 ou la revendication 2 ;
fournir le au moins un mélange échantillon à un dispositif microfluidique, le mélange échantillon comprenant une enzyme, un substrat enzymatique, un composé à tester et un produit ;
appliquer une pression de vide au mélange échantillon ;
faire circuler le mélange échantillon le long d'un microcanal du dispositif microfluidique en réponse à la pression appliquée ;
séparer le produit et le substrat enzymatique sur la base d'une différence de mobilité électrophorétique entre le produit et le substrat enzymatique ;
détecter le produit séparé et le substrat enzymatique, et
déterminer l'activité d'inhibition enzymatique du composé sur la base de la détection du produit séparé et du substrat enzymatique.

6. Procédé selon la revendication 5, dans lequel la fourniture du au moins un mélange échantillon au dispositif microfluidique comprend la circulation du mélange échantillon depuis un puits de la première pluralité de puits jusque dans le microcanal via un élément capillaire, l'élément capillaire étant connecté de manière opérationnelle à et en communication fluidique avec le microcanal.

7. Procédé selon la revendication 5, dans lequel le au moins un mélange échantillon est sélectionné parmi la première pluralité de puits, la première plaque multipuits comportant une pluralité de colonnes de puits, dans lequel chaque puits de chaque colonne comprend une seule enzyme.

8. Procédé selon la revendication 5, dans lequel la préparation d'au moins un mélange échantillon à l'aide du kit selon la revendication 1 ou 2 comprend les étapes consistant à : décongeler les première et seconde plaques multipuits, les première et seconde plaques multipuits ayant été conservées congelées ; ajouter un tampon de reconstitution à chaque puits de la première pluralité de puits ; ajouter un composé à chaque puits de la première pluralité de puits ; ajouter un substrat enzymatique de chacun de la seconde pluralité de puits à chaque puits correspondant de la première pluralité de puits ; et ajouter un tampon de terminaison à chacun de la première pluralité de puits.
